**Europäisches Patentamt**

**European Patent Office**

**Office européen des brevets**

(19)

(11) Veröffentlichungsnummer: **0 180 664**
**B1**

# (12) EUROPÄISCHE PATENTSCHRIFT

(45) Veröffentlichungstag der Patentschrift :
04.05.88

(51) Int. Cl.⁴: **B 01 D 19/04, C 12 N   1/38//**
**C12N1/18**

(21) Anmeldenummer : 84114864.6

(22) Anmeldetag : 06.12.84

(54) **Entschäumer für die Hefefermentation.**

(30) Priorität : 24.09.84 DE 3434984

(43) Veröffentlichungstag der Anmeldung :
14.05.86 Patentblatt 86/20

(45) Bekanntmachung des Hinweises auf die Patenterteilung : 04.05.88 Patentblatt 88/18

(84) Benannte Vertragsstaaten :
AT BE CH DE FR GB IT LI NL

(56) Entgegenhaltungen :
EP-A- 0 019 173
EP-A- 0 097 786
DE-A- 2 410 155
FR-A- 2 074 419
GB-A-   775 483
US-A- 3 920 559

(73) Patentinhaber : Henkel Kommanditgesellschaft auf
Aktien
Postfach 1100 Henkelstrasse 67
D-4000 Düsseldorf-Holthausen (DE)

(72) Erfinder : Schmid, Karl, Dr.
Stifterstrasse 10
D-4020 Mettmann (DE)
Erfinder : Schindler, Joachim, Dr.
Am Eichelkamp 156
D-4010 Hilden (DE)
Erfinder : Asbeck, Adolf
Am Langen Weiher 51
D-4000 Düsseldorf 13 (DE)

Jouve, 18, rue St-Denis, 75001 Paris, France

## Beschreibung

Die Herstellung von Hefen, insbesondere Backhefe, erfolgt bekanntlich im großen Maßstab durch Fermentation von Melasse. Dabei kann es insbesondere in neuzeitlichen, auf hohe Effektivität ausgelegten Fermentationsanlagen zu einer erheblichen, den Betriebsablauf störenden Schaumbildung kommen. Diese Schaumbildung muß durch Zusatz von Entschäumern unterdrückt werden.

In den letzten Jahren wurde nun von den Hefeproduzenten moniert, daß die Backhefe bei Lagerung ihre Konsistenz ändert und schließlich weich bis sogar flüssig und damit unverkäuflich wird. Für die verminderte Hefequalität wird von der Hefeindustrie der steigende Tensidanteil in der Melasse verantwortlich gemacht.

Die Anmelderin hat festgestellt, daß die Konsistenz der Backhefe von der Art des Antischaummittels beeinflußt wird und daß alle bisher auf dem Markt befindlichen bzw. für diesen Zweck vorgeschlagenen Hefeentschäumer zu einem mehr oder weniger schnellen Erweichen der Hefe führen.

Es bestand daher großes Interesse an einem Entschäumer, der bereits in geringer Dosierung eine störende Schaumentwicklung bei der Hefefermentierung verhindert eine möglichst geringe Wachstumshemmung auf die Hefekulturen ausübt und innerhalb einer angemessenen Lagerzeit der fertigen Hefe nicht zu einer Erweichung bzw. Verflüssigung führt. Diese Aufgabe wird durch die vorliegende Erfindung gelöst.

Gegenstand der Erfindung ist die Verwendung von Polyglykolethern linearer, primärer, gesättigter und einfach ungesättigter Alkohole mit 16 bis 18 Kohlenstoffatomen, Ethylenglykolethergruppen und/oder Propylenglykolethergruppen, als Entschäumer bei der Hefefermentation.

Die erfindungsgemäß zu verwendenden Polyglykolether leiten sich von Fettalkoholen natürlichen oder synthetischen Ursprungs mit 16 bis 18 Kohlenstoffatomen ab, insbesondere von Gemischen aus Oleylalkohol und Cetylalkohol. Auch Myristyl- und Stearylalkohol können im Gemisch mit Oleylalkohol und Cetylalkohol vorliegen, jedoch sollte deren Anteil jeweils 10 Gew.-% nicht überschreiten. Besonders geeignet sind Polyglykolether, in denen die Alkoholkomponente zu 30 bis 80 % aus Oleylalkohol besteht. Höher ungesättigte Alkohole sind ungeeignet und sollten vorzugsweise abwesend sein.

Die Anzahl der Polyglykolethergruppen beträgt im Durchschnitt 1 bis 4, vorzugsweise 1 bis 3. Wie bei technischen Alkoxylierungsprodukten üblich, stellt diese Zahlenangabe einen statistischen Mittelwert dar, d. h. die Alkoxylierungsprodukte können entsprechend einer statistischen Verteilung auch untergeordnete Anteile an höher bzw. niedriger alkoxylierten einschließlich unsubstituierten Alkoholen enthalten. Die Glykolethergruppen können aus Ethylenglykolethergruppen (EO) oder Propylenglykolethergruppen (PO) bestehen oder auch gemeinsam vorliegen. Vorzugsweise bestehen sie aus EO-Gruppen, wobei deren Anzahl durchschnittlich 1 bis 3 beträgt.

Als besonders wirksam haben sich Polyglykolether erwiesen, die sich von Gemischen aus Oleylalkohol und Cetylalkohol im Gewichtsverhältnis 3 : 1 bis 1 : 1 ableiten und durchschnittlich 2 EO-Gruppen aufweisen.

Gegenstand der Erfindung ist weiterhin ein Verfahren zum Entschäumen wäßriger Hefefermentbrühen, das dadurch gekennzeichnet ist, daß man der Fermentbrühe 0,005 bis 1 g/l an Polyglykolethern der vorstehend beschriebenen Art zusetzt. Vorzugsweise betragen die Einsatzmengen 0,01 bis 0,2 g/l.

Die erfindungsgemäß zu verwendenden Entschäumer sind allen bisher bekannten und insbesondere für den Einsatz in Fermentbrühen speziell entwickelten Produkten hinsichtlich optimaler Entschäumerwirkung, minimaler Wachstumshemmung und hoher Beständigkeit der behandelten Hefen gegen Erweichen und Verflüssigung überlegen. Selbst bei einer 10 fachen Überdosierung über die für eine ausreichende Schaumunterdrückung erforderliche Einsatzmenge hinaus stellen sich keine nennenswerten negativen Effekte ein.

## Beispiele

Als Entschäumer wurden die folgenden Produkte getestet :

I = $C_{16}$-$C_{18}$-Fettalkohol + 2 EO (35 % Cetyl-, 60 % Oleyl-, 5 % Stearylalkohol, JZ = 53)
II = $C_{16}$-$C_{18}$-Fettalkohol + 3 EO (Alkoholbasis wie I)
III = $C_{16}$-$C_{18}$-Fettalkohol + 1 PO (Alkoholbasis wie I)

Zum Vergleich wurden folgende Stoffe mit bekannter Entschäumerwirkung herangezogen. (Die Abkürzung M bedeutet Molekulargewicht.)

$V_1$  Ölsäure + 1,2 EO
$V_2$  Ölsäuremethylester
$V_3$  Glycerinmonooleat
$V_4$  Ricinusöl + 5,5 EO
$V_5$  Polypropylenglykol (M = 2 020)
$V_6$  Polypropylenglykol (M = 3 800) + 22 Gew.-% EO

2

$V_7$ gesättiger $C_{12}$-$C_{18}$-Fettalkohol + 2 EO + 4 PO
$V_8$ gesättiger $C_{12}$-$C_{18}$-Fettalkohol + 5 EO + 13 PO
$V_9$ gesättiger $C_{12}$-$C_{18}$-Fettalkohol + 14 EO + 54 PO
$V_{10}$ gesättiger $C_{12}$-$C_{18}$-Fettalkohol + 2 EO
$V_{11}$ Oleylalkohol (95 %) + Linoleylalkohol (5 %) + 2 EO
$V_{12}$ handelsüblicher Fermentationsentschäumer A
$V_{13}$ handelsüblicher Fermentationsentschäumer B.

Bei den handelsüblichen Entschäumern handelt es sich um die Produkte Struktol ® J 673 (A) und Struktol ® KG 11 II (B) der Firma Schill & Seilacher, Hamburg.

Die Versuchsdurchführung erfolgte in Fermentationsbehältern, in die in Bodennähe feinverteilte Luft eingeleitet wurde. Die fermentationsbrühen bestanden aus verdünnter, mit einem handelsüblichen Backhefestamm der Gattung Saccharomyces cerevisiae geimpfter Melasse und enthielten die üblichen Wuchs- und Zusatzstoffe. Es wurde jeweils so viel Entschäumer zugesetzt, so daß sich keine geschlossene Schaumdecke bilden konnte. Weiterhin wurde die durch Zusatz von 0,1 Gew.-% bzw. 1 Gew.-% Entschäumer bewirkte Wachstumshemmung bewertet. Außerdem wurde die Festigkeit der gewonnenen Preßhefe in Abhängigkeit von der Lagerzeit (Lagerung bei 25 °C) bestimmt. Die Ergebnisse sind in der Folgenden Tabelle zusammengestellt.

In der Spalte « Wachstumshemmung » bedeutet — keine Hemmung, (—) leichte Hemmung und + starke Hemmung

| Probe | Dosierung g/l | Wachstumshemmung 0,1 % | 1 % | Erweichung nach Tagen |
|---|---|---|---|---|
| I | 0,02 | – | – | 28 |
| II | 0,3 | – | – | 13 |
| III | 0,07 | – | – | 20 |
| $V_1$ | 0,59 | – | – | 5 |
| $V_2$ | 0,05 | – | (–) | sofort |
| $V_3$ | 0,59 | – | + | 8 |
| $V_4$ | 0,11 | + | + | sofort |
| $V_5$ | 0,27 | – | – | sofort |
| $V_6$ | 0,07 | – | – | sofort |
| $V_7$ | 0,61 | – | – | 11 |
| $V_8$ | 0,41 | + | + | sofort |
| $V_9$ | 0,22 | + | + | 20 |
| $V_{10}$ | 0,46 | – | + | sofort |
| $V_{11}$ | 1,1 | + | + | 14 |
| $V_{12}$ | 0,5 | – | (–) | 10 |
| $V_{13}$ | 0,8 | – | – | 3 |

Bei den Proben $V_1$, $V_3$, $V_7$, $V_{12}$ und $V_{13}$ war keine Wachstumshemmung und eine mittlere Beständigkeit der Preßhefe zu beobachten, jedoch liegen die notwendigen Anwendungskonzentrationen relativ hoch. Die übrigen Vergleichsproben führten zu Wachstumshemmungen und/oder einem sofortigen Weichwerden bzw. Verflüssigen der Preßhefe.

Eine Spitzenstellung nimmt das Produkt I ein, das bereits bei einer Konzentration von 0,02 g/l eine störende Schaumentwicklung unterbindet, keine Wachstumshemmung bewirkt und zu besonders beständigen Preßhefen führt. Auch bei einer 10 fachen Einsatzmenge (0,2 g/l) dieses Zusatzes waren noch keine negativen Einflüsse zu beobachten. Die Erweichung der Hefe trat bei dieser Einsatzmenge zwar nach 21 Tagen ein, jedoch stellt auch dieser Wert noch einen Spitzenwert dar.

## Patentansprüche

1. Verwendung von Polyglykolethern linearer, primärer, gesättigter und einfach ungesättigter Alkohole

3

mit 16 bis 18 Kohlenstoffatomen, enthaltend durchschnittlich 1 bis 4 Ethylenglykolethergruppen und/oder Propylenglykolethergruppen, als Entschäumer bei der Hefefermentation.

2. Verwendung von Polyglykolethern gemäß Anspruch 1, worin die Alkolholkomponente zu 30 bis 80 Gew.-% Oleylalkohol besteht.

3. Verwendung von Polyglykolethern gemäß Anspruch 1 und 2, enthaltend durchschnittlich 1 bis 3 Ethylenglykoletergruppen.

4. Verwendung von Polyglykolethern gemäß Anspruch 1 bis 3, worin die Alkoholkomponente aus einem Gemisch von Oleylalkohol und Cetylalkohol im Gewichtsverhältnis 3 : 1 bis 1 : 1 besteht und die durchschnittliche Zahl der Ethylenglykolethergruppen 2 beträgt.

5. Verfahren zum Entschäumen wäßriger Hefefermentbrühen, dadurch gekennzeichnet, daß man der Fermentbrühe 0,005 bis 1 g/l, vorzugsweise 0,01 bis 0,2 g/l, eines in den Ansprüchen 1 bis 4 definierten Polyglykolethers zusetzt.

## Claims

1. The use of polyglycol ethers of linear, primary, saturated and monounsaturated $C_{16}$-$C_{18}$ alcohols containing on average from 1 to 4 ethylene glycol ether groups and/or propylene glycol ether groups as defoamers in the fermentation of yeast.

2. The use of polyglycol ethers as claimed in Claim 1 in which from 30 to 80 % by weight of the alcohol component consists of oleyl alcohol.

3. The use of polyglycol ethers as claimed in Claims 1 and 2 containing on average from 1 to 3 etylene glycol ether groups.

4. The use of polyglycol ethers as claimed in Claims 1 to 3 in which the alcohol component consists of a mixture of oleyl alcohol and cetyl alcohol in a ration by weight of from 3 : 1 to 1 : 1 and the average number of ethylene glycol ether groups amounts to 2.

5. A process for defoaming aqueous yeast ferment broths, characterized in that from 0.005 to 1 g/l and preferably from 0.01 to 0.2 g/l of a polyglycol ether according to Claims 1 to 4 is added to the ferment broth.

## Revendications

1. Utilisation de polyglycol-éthers d'alcools linéaires, primaires, saturés et à insaturation simple contenant 16 à 18 atomes de carbone et comportant, en moyenne, 1 à 4 groupes éthylène-glycol-éther et/ou propylène-glycol-éther, comme agents antimousses lors de la fermentation des levures.

2. Utilisation de polyglycol-éthers selon la revendication 1, dans laquelle le composant alcool est constitué pour 30 à 80 % en poids d'alcool oléylique.

3. Utilisation de polyglycol-éthers selon les revendications 1 et 2, contenant, en moyenne, 1 à 3 groupes éthylène-glycol-éther.

4. Utilisation de polyglycol-éthers selon les revendications 1 à 3, dans laquelle le composant alcool est constitué d'un mélange d'alcool oléylique et d'alcool cétylique dans le rapport pondéral de 3 : 1 à 1 : 1, tandis que le nombre moyen de groupes éthylène-glycol-éther est de 2.

5. Procédé pour l'inhibition de la formation de mousse dans des bouillons aqueux de fermentation de levures, caractérisé en ce que, au bouillon de fermentation, on ajoute 0,005 à 1 g/l, de préférence, 0,01 à 0,2 g/l d'un polyglycoléther selon les revendications 1 à 4.